(19) Europäisches Patentamt
European Patent Office
Office européen des brevets

(11) **EP 4 350 703 A1**

(12) **EUROPEAN PATENT APPLICATION**

(43) Date of publication:
**10.04.2024 Bulletin 2024/15**

(21) Application number: **22315233.1**

(22) Date of filing: **07.10.2022**

(51) International Patent Classification (IPC):
**G16H 15/00** (2018.01)      **G16H 20/17** (2018.01)
**G16H 10/60** (2018.01)      **A61M 5/14** (2006.01)

(52) Cooperative Patent Classification (CPC):
**G16H 20/17; G16H 15/00;** A61M 5/14; G16H 10/60;
G16H 40/67

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **SANOFI**
**75008 Paris (FR)**

(72) Inventor: **The designation of the inventor has not yet been filed**

(74) Representative: **Sanofi Aventis Deutschland
GmbH
Industriepark Höchst
Geb. K703
65926 Frankfurt am Main (DE)**

(54) **METHOD, APPARATUS AND DEVICES FOR DETERMINING AN ABSOLUTE TIME OF ONE OR MORE RECORDED DOSE EVENTS PERFORMED WITH A MEDICAL DEVICE**

(57)      Method for determining an absolute time, $T_{Dose}(e)$, of one or more recorded dose events ($e_1$ to $e_z$) performed with a medical device, wherein $e$ characterizes one particular dose event, wherein the one or more dose events are recorded in a dose event record, wherein at least one part of the dose event record is received (S100) by or receivable by a receiving unit (71), and wherein $T_{Dose}(e)$ is determined (S104) based on at least one time information from a recording unit (11), by which the dose event was recorded for the dose event record, and at least one time information from the receiving unit (71).

Fig. 1

**Description**

Background

In medicament-based therapies, it often is important to determine not only the quantity of medicament that is administered to the patient, but also the exact time at which the administration happened.

For example, looking at diabetes treatment, if a patient takes too much insulin in a certain period of time, this might lead to hypoglycaemia with severe consequences for the patient. Thus, in order not to exceed a critical quantity of insulin in a certain period, e.g. a critical daily quantity, the patient needs to keep track of the administered doses and the times of administration.

Usually an insulin dose is administered with a medicament delivery device. Such a medicament delivery device can provide the functionality of recording the quantity of medicament and time information at which the medicament had been administered, for example in a dose record or dose log. Alternatively, or additionally, there are add-ons for medicament delivery devices which can provide this functionality.

Typically, the time information is acquired with a timer, e.g. a real time clock, within the medicament delivery device or within the add-on.

Based on the information acquired with the timer, a dose time, $T_{Dose}(e)$, at which a dose e was administered is calculated, in order to generate a dose log based on which the patient or the health care professional can monitor the dose administrations.

This means that according to the prior art the determination of dose time entirely relies on the accuracy of the real time clock within the medicament device or within the add-on.

Assuming that this real time clock can never be perfectly accurate there always will be a slight deviation between the registered time and the real time at which a dose was administered. This deviation or drift increases with time, and so will inaccuracies in the dose log. This is potentially dangerous for patients.

Summary

It is an object of the present disclosure to provide improvements relating to the determination of an absolute time of a recorded dose event.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. A drug or medicament may be used for a limited duration, or on a regular basis for chronic disorders. Further explanations regarding to "drug" or "medicament" can be found in the last part of the description of the exemplary embodiments.

In the following the term "distal" refers to a direction of a medical device, a medicament delivery device or a medicament container in which medicament is discharged. Accordingly, the term "proximal" refers to the opposite direction. As regards pen-shaped medicament delivery devices, the term "distal" refers to the direction towards the injection site and/or the tip of an injection needle of the device. Accordingly, the term "proximal" refers to the direction pointing away from the injection site and/or the tip of an injection needle of the device.

Herein, the term "absolute time" refers to a standard time, e.g. to the so-called Coordinated Universal Time or UTC, which is the primary time standard for the regulation of time and clocks worldwide. The standard time may be a time easily interpreted by users.

Herein, the expression "dose event" relates to any action or event that can be performed with a medical device configured to contain a medicament, e.g. a medicament delivery device. Among others, such an action or event can be setting and/or selecting an amount, i.e. a quantity of medicament, to be output from the medical device, e.g. ejected or expelled. The quantity may be selected by a user action. Alternatively or additionally, the expression "dose event" relates to any action or event that can be performed with a medical device in order to output medicament from the medical device, e.g. expelling and/or ejecting a quantity medicament (which may have been selected previously), which might be injected to a patient's body, such as via a needle. It should be understood that the medicament can be administered in other ways, too.

A "dose" may be a part or the entire amount/quantity of medicament that is set, selected, output, expelled and/or ejected from the medical device in a single process. In particular, this is not limited to a unit-based quantification of the medicament. If, however, a dose may be quantified in increments only, a "dose" may comprise one or more than one increment.

Herein, the expressions "time" and "point in time" may be used synonymously and describe time information relating to certain actions. This may be the start of a transmission of at least a part of a dose event record from a recording unit to a receiving unit or the end of said transmission, wherein the start may be indicated by time information from the recording unit while the end may be indicated by time information from the receiving unit.

The object is achieved by the subject-matter disclosed herein, for example.by the subject-matter defined in the appended independent claims. Advantageous refinements and developments are subject to the dependent claims and/or set forth in the description below.

One aspect of the present disclosure relates to a method for determining an absolute time, $T_{Dose}(e)$, of one or more recorded dose events ($e_1$ to $e_z$), performed with a medical device, wherein e characterizes one particular dose event, and wherein the index z is a positive integer greater than or equal to one.

The one or more dose events are recorded in a dose event record comprising information characterizing the one or more dose events contained in the dose event record.

The dose event record may be a continuous record of all dose events performed with the medical device, e.g. while delivering medicament from one reservoir or a plurality of reservoirs.

At least one part of the dose event record is received by or receivable by a receiving unit. The at least one part of the dose event record may comprise the one or more dose events $e_1$ to $e_z$. The dose events may be consecutive dose events or a selection of dose event recorded in the dose event record..

The at least one part of the dose event record may comprise information relating to at least one of, an arbitrary selected plurality of, or all of: a unique dose event identifier, a dose quantity and/or a dose event related time information, $T_{Stamp}(e)$, of the recording unit which is indicative for the time at which the dose event e was recorded in the at least one part of the dose event record.

The dose event identifier may be unique, e.g. within the dose event record or overall. The dose event identifier may be a unique number, text or combination of signs, based on which one particular dose event can be identified unequivocally. The dose quantity may be a quantity or amount of medicament which is set, selected, or output, e.g. ejected, expelled or the like, in a single dose event.

The absolute time, $T_{Dose}(e)$, is determined based on at least one time information from a recording unit, by which the dose event was recorded for the dose event record, and at least one time information from the receiving unit.

In one embodiment, the time information from the recording unit may comprise one of, an arbitrarily selected plurality of, or all of $T_{stamp}(e)$, $T_{RTC\_trans}$, and $T_{RTC\_FDE}$. $T_{Stamp}(e)$ may be dose event related time information of the recording unit which is indicative for the time at which the dose event e was recorded in the dose event record. $T_{RTC\_trans}$ may be a transmission related time information of the recording unit which is indicative for the time at which the at least one part of the dose event record containing the dose event e is transmitted to be received by the receiving unit. $T_{RTC\_FDE}$ may be a first dose event time information of the recording unit which is indicative for the time at which a first dose event is recorded by the recording unit in the dose event record.

In one embodiment, the time information from the recording unit may comprise one of, or all of $T_{stamp}(e)$, and $T_{RTC\_trans}$, e.g. only. In other words, the time information from the recording unit may not comprise $T_{RTC\_FDE}$.

The time information from the receiving unit may comprise a time, $T_{Client}$, of the receiving unit which is indicative for the time at which the at least one part of the dose event record is received by the receiving unit.

The method may further comprise the step of evaluating the at least one part of the dose event record with respect to an occurrence of an irregularity in a time information contained in the at least one part of the dose event record, that is received by the receiving unit.

The evaluation may be based only on the information stored in the dose event record, e.g. $T_{stamp}(e)$, and/or time information from the recording unit, e.g. $T_{RTC\_trans}$. Alternatively, the evaluation may be based on information stored in the dose event record, and/or time information from the recording unit, in combination with time information from the receiving unit, e.g. $T_{Client}$.

The evaluation of an occurrence of an irregularity may provide the advantage that such an irregularity may be considered when determining the absolute time, $T_{Dose}(e)$, of one or more recorded dose events, $e_1$ to $e_z$. That is, different determination functions may be used for determining $T_{Dose}(e)$ for different dose events, depending on when the dose event was recorded with respect to the irregularity. Advantageously, this may improve the accuracy of the determination.

The recording unit may comprise a timer configured to provide time information for the recording unit, the time information being $T_{RTC}$. If not reset, the value of $T_{RTC}$ increases continuously. The timer of the recording unit may comprise a real time clock.

In one embodiment, the initial value of $T_{RTC}$ may be to set according to an absolute time, e.g. the Coordinated Universal Time, UTC, during manufacturing of the recording unit. That is, the initial value of $T_{RTC}$ may be set to correspond to an absolute time when the timer starts to run, e.g. at the moment when the recording unit is connected to an energy source during assembly.

In one embodiment, the value of $T_{RTC}$ at the moment when a first dose event is recorded in the dose event record may be stored as $T_{RTC\_FDE}$. In one embodiment, the value of $T_{RTC}$ at the moment when a first dose event is recorded in the dose event record may not be stored

In one embodiment, the value of $T_{RTC}$ may be set to zero when a first dose event is recorded in the dose event record. In one embodiment, $T_{RTC}$ may be set to zero after at least one part of the dose event record is transmitted to be received by the receiving unit and/or after the transmitted part is received by the receiving unit. In one embodiment,

$T_{RTC}$ may be set to zero each time after a part of the dose event record is transmitted to be received by the receiving unit and/or after the transmitted part of the dose event record is received by the receiving unit. Setting $T_{RTC}$ to zero after a part of the dose event record is transmitted to be received by the receiving unit may provide the advantage that time deviations between the timer of the recording unit and the accurate real time, e.g. the UTC, may have less negative influence on the accuracy of the determination of $T_{Dose}(e)$.

The time information $T_{RTC}$ may be in a format which is intuitively readable by a human, e.g. the UTC format. Alternatively, $T_{RTC}$ may be in a format which is not intuitively readable by a human, e.g. the UNIX time (EPOCH time). Such a format might be advantageous for processing the time information.

The irregularity may be a reset event of the recording unit at which the timer of the recording unit is reset resulting in a change of $T_{RTC}$ to a default value of $T_{RTC}$. The default value may be any predefined value different from zero. Alternatively, the default value may be zero.

In an embodiment, the irregularity may be a temporary stop or pause of $T_{RTC}$.

In one embodiment, the occurrence of an irregularity may be evaluated based on the time information from the receiving unit and/or based on information stored in the dose event record and/or based on the time information from the recording unit. The occurrence of an irregularity may be evaluated based on a detection of a change, e.g. a step change, in a variable $\Delta$, which is indicative for a time deviation between the recording unit and the receiving unit.

In one embodiment, $\Delta$ may be determined after a time, *m*, at which at least one part of the dose event record is transmitted to be received by the receiving unit and after a time, *p*, at which the at least one part of the dose event record is received by the receiving unit. $\Delta$ may be determined based on time information from the recording unit and time information from the receiving unit, e.g. $T_{RTC\_m}$ and $T_{Client\_p}$. $T_{RTC\_m}$ may be a transmission related time information of the recording unit which is indicative for the time, *m*, at which the at least one part of the dose event record is transmitted to be received by the receiving unit, and $T_{Client\_p}$ may be a time information from the receiving unit which is indicative for the time, *p*, at which the at least one part of the dose event record is received by the receiving unit.

In one embodiment, $\Delta$ may be determined according to the equation:

$$\Delta = T_{Client\_p} - T_{RTC\_m}.$$

In one embodiment, $\Delta$ may be determined each time a part of the dose event record is received by the receiving unit. Alternatively, $\Delta$ may be determined less often, e.g. only every second, third fourth, fifth, tenth, twentieth or fiftieth time at which a part of the dose event record is received by the receiving unit.

The values for one, several, or every determined $\Delta$ may be stored in a memory unit as $\Delta_{pre}$, for further processing at a later point in time. In one embodiment, if $\Delta_{pre}$ is determined more than once, it either may be updated every time, such that only the most recent $\Delta_{pre}$ is stored.

Alternatively, more than one or all determined $\Delta_{pre}$ may be stored in the memory with a continuously increasing index *i*, as $\Delta_{pre\_i}$.

Apart from slight drifts due to design tolerances of electronic components of the recording unit and/or the receiving unit, $\Delta$ may remain approximately constant, when there is no irregularity in the dose event record. Hence, in one embodiment, the occurrence of an irregularity in the dose event record may be evaluated based on a detection of a change, e.g. a step change, in the variable $\Delta$.

In one embodiment, this change may be detected by comparing the most recent value of $\Delta$ with one of the stored $\Delta_{pre\_i}$ or the stored $\Delta_{pre}$. For this comparison and the resulting evaluation of an occurrence of an irregularity in the dose event record, it is advantageous if more than one $\Delta_{pre\_i}$ is compared with $\Delta$.

In one embodiment, if no part of the dose event record has been transmitted before such that there is no $\Delta_{pre\_i}$ or $\Delta_{pre}$ which could be used for the determination of the change, the determination that there is a reset of $T_{RTC}$ in the determination period may be made based on information stored in the dose event record. For example, if $T_{Stamp}(e)$ for subsequent dose events does not increase, this indicates a reset of $T_{RTC}$ between these dose events.

In one embodiment, the occurrence of an irregularity may be evaluated based on ...

The method may comprise the step of defining a determination period in at least one part of the dose event record, which is received by the receiving unit. The determination period may comprise one or more dose events, $e_i$ to $e_k$, for which $T_{Dose}(e)$ is to be determined, wherein $k \geq i \geq 1$.

The determination period may be defined based on the dose event identifier of the dose events, $e_i$ to $e_k$, recorded in the at least one part of dose event record for which $T_{Dose}(e)$ should be determined.

In one embodiment, $e_i$ to $e_k$ may be dose events for which $T_{Dose}(e)$, has not been determined before. Alternatively, at least one of $e_i$ to $e_k$ may be a dose event for which $T_{Dose}(e)$ had already been determined once or several times before.

In one embodiment, the determination period may be selected by a user, e.g. via a user interface operatively connected to the receiving unit.

In one embodiment, the determination period may extend over the whole length of the received part of the dose event record.

The absolute time, $T_{Dose}(e)$, of a particular dose event, $e$, of the one or more dose events, $e_i$ to $e_k$, within the determination period, may be determined using a determination function.

The determination function $f$ may be a function of the time information from the recording unit and the time information from the receiving unit.

In one embodiment, the determination function $f$ may be such that $T_{Dose}(e) = f(T_{Client}, T_{RTC\text{-}trans}, T_{Stamp}(e))$.

In one embodiment, the determination function $f$ may be a function of the time information from the receiving unit and a difference of different time information from the recording unit, e.g. $T_{RTC\_trans} - T_{stamp}(e)$, such that $T_{Dose}(e) = f(T_{Client}, (T_{RTC\_trans} - T_{Stamp}(e)))$.

In one embodiment, the determination function $f$ may be a function of a difference between the time information from the receiving unit and the time information from the recording unit, e.g. $T_{RTC\_trans} - T_{Stamp}(e)$, such that $T_{Dose}(e) = f(T_{Client} - (T_{RTC\_trans} - T_{Stamp}(e)))$.

In one embodiment, the determination function $f$ may be such that $T_{Dose}(e) = T_{Client} - (T_{RTC\_trans} - T_{Stamp}(e))$.

In one embodiment, time information of the recording unit may comprise a transmission related time information, $T_{RTC\_m}$, of the recording unit which is indicative for a first point in time, $m$, at which at least a first part of the dose event record had been transmitted to be received by the receiving unit. If there had not been such a transmission of at least a first part of the dose event record before, $T_{RTC\_m}$ may be set to the default value of $T_{RTC}$. For example, $T_{RTC\_m}$ may be set to zero.

In one embodiment, the time information of the recording unit may comprise a transmission related time information, $T_{RTC\_m+1}$, of the recording unit which is indicative for a second point in time, $m + 1$, at which at least a second part of the dose event record is transmitted to be received by the receiving unit. In one embodiment, the determination period may extend between $T_{RTC\_m}$ and $T_{RTC\_m+1}$.

In one embodiment, the time information from the receiving unit may comprise a time information, $T_{Client\_p}$, of the receiving unit which is indicative for a third point time, $p$, at which the first part of the dose event record had been received by the receiving unit. The third point in time may be earlier than the second point in time.

In one embodiment, the time information from the receiving unit may comprise a time information, $T_{Client\_p+1}$, of the receiving unit which is indicative for a fourth point in time, $p + 1$, at which the second part of the dose event record is received by the receiving unit. If, at the third point in time, no part of the dose event record had been received by the receiving unit, the mentioned second part of the dose event record may be the at least one part of the dose event record, as described before.

In one embodiment, the determination function for the particular dose event may be selectable or selected from a plurality of different determination functions depending on the occurrence of an irregularity in the time information of the at least one part of the dose event record during the determination period.

In one embodiment, a determination function, $g$, may be selected if the determination period does not contain any irregularity. Likewise, $g$ may be selected if the dose event for which $T_{Dose}(e)$ is to be determined was recorded after a last reset of $T_{RTC}$ in the determination period. In this context, the last reset of $T_{RTC}$ may be a reset of $T_{RTC}$ in the determination period after which there is no subsequent reset of $T_{RTC}$ in the determination period. In other words, if there is no reset event between the last reset of $T_{RTC}$ and the point in time at which the at least one part of the dose event record is transmitted to be received by the receiving unit.

In one embodiment, the determination function, $g$, may be selected if the determination period starts with a reset of $T_{RTC}$.

In one embodiment, a determination function, $h$, may be selected if the determination period starts after a time, $p$, at which at least a part of the dose event record had been received by the receiving unit and if the dose event, $e$, for which $T_{Dose}(e)$ is to be determined was recorded before a first reset of $T_{RTC}$ within the determination period.

In one embodiment, a determination function, $i$, may be selected, if neither the conditions for selecting $g$ or $h$ are fulfilled. For example, $i$ may be selected if the dose event, $e$, for which $T_{Dose}(e)$ is to be determined was recorded between an $n$-th reset of $T_{RTC}$ and an $(n + 1)$-th reset of $T_{RTC}$ in the determination period, with $n \geq 1$.

The determination function $i$ may be a function of $T_{stamp}(e)$, $T_{User}(e_{other})$ and $T_{Stamp}(e_{other})$.

$T_{Stamp}(e)$ may be a dose event related time information of the recording unit which is indicative for the time at which the dose event, $e$, was recorded in the dose event record, as described before.

$T_{User}(e_{other})$ may be a user entered time of one other dose event, $e_{other}$, which was recorded in the same portion of the determination period as the dose event e, for which $T_{Dose}(e)$ is to be determined. In this context, "same portion" means that there is no irregularity or reset of $T_{RTC}$ in the dose event record between the dose event e and the dose event $e_{other}$. For example, if the dose event, e, for which $T_{Dose}(e)$ is to be determined was recorded between an $n$-th reset of $T_{RTC}$ and an $(n + 1)$-th reset of $T_{RTC}$ in the determination period, with $n \geq 1$, the other dose event, $e_{other}$, must have been recorded between the $n$-th reset event and the $n + 1$-th reset event in the determination period, as well.

$T_{Stamp}(e_{other})$ may be a dose event related time information of the recording unit which is indicative for the time at which the other dose event, $e_{other}$, was recorded in the dose event record.

In one embodiment, the determination functions $g$, $h$, and $i$ may be different determination functions.

In one embodiment, at least two of the determination functions $g$, $h$, and $i$ may be equal determination functions.

The absolute time, $T_{Dose}(e)$, of a particular dose event, $e$, recorded in the dose event record may be determined according to one of the determination functions below:

$$T_{Dose}(e) = g(T_{Client} - (T_{RTC\_trans} - T_{Stamp}(e))),$$

and/or

$$T_{Dose}(e) = h(\Delta_{pre} + T_{Stamp}(e)),$$

and/or

$$T_{Dose}(e) = i(T_{User}(e_{other}) - T_{Stamp}(e_{other}) + T_{Stamp}(e)).$$

In one embodiment, the determination functions $g$, $h$, and $i$ may be functions of the variables and/or expressions specified in the brackets.

In one embodiment, according to the determination function $g$:

$$T_{Dose}(e) = T_{Client} - (T_{RTC\_trans} - T_{Stamp}(e)).$$

In one embodiment, according to the determination function $h$:

$$T_{Dose}(e) = \Delta_{pre} + T_{Stamp}(e).$$

In one embodiment, according to the determination function $i$:

$$T_{Dose}(e) = T_{User}(e_{other}) - T_{Stamp}(e_{other}) + T_{Stamp}(e).$$

In one embodiment, a determination function, $j$, may be selected, if the dose event for which $T_{Dose}(e)$ is to be determined was recorded between a first dose event recorded in the dose event record and a first irregularity in the determination period. In other words, referring to the first dose event recorded in the dose event record, it is appreciated that the determination function j may be selected if the determination period starts with the first dose event recorded in the dose event record.

In one embodiment, a determination function, $k$, may be selected, if there is no reset of $T_{RTC}$ in the determination period.

In one embodiment, a determination function, $l$, may be selected, if the determination period starts after an earlier point in time, $p$, at which at least one part of the dose event record had been successfully received by the receiving unit, further if $T_{RTC}$ is not at its default value at the start of the determination period, and further if the dose event, $e$, for which $T_{Dose}(e)$ is to be determined was recorded before a first reset of $T_{RTC}$ within the determination period. The first reset of $T_{RTC}$ is a reset of $T_{RTC}$ in the determination period before which there is no previous reset of $T_{RTC}$ in the determination period.

In one embodiment, the determination functions $g$, $j$, $k$ and $l$ may be different determination functions.

In one embodiment, at least two of the determination functions $g$, $j$, $k$, and $l$ may be equal determination functions.

In one embodiment, one of, an arbitrarily selected plurality of, or all of $T_{RTC}$, $T_{RTC\_FDE}$, $T_{RTC\_trans}$, $T_{RTC\_m}$, $T_{RTC\_m+1}$, $T_{stamp}(e)$, $T_{Stamp}(e_{other})$, $T_{User}(e_{other})$, $T_{Client}$, $T_{Client\_p}$, and $T_{Client\_p+1}$ may be determined by an absolute time, e.g. the Coordinated Universal Time, UTC.

In one embodiment, $T_{Client}$, $T_{Client\_p}$ and $T_{Client\_p+1}$ of the receiving unit may be indicative for a point in time at which at least one part of the dose event record is received by the receiving unit. In one embodiment, one of, an arbitrarily selected plurality of, or all of $T_{RTC}$, $T_{RTC\_FDE}$, $T_{RTC\_trans}$, $T_{RTC\_m}$, $T_{RTC\_m+1}$, $T_{stamp}(e)$, $T_{Stamp}(e_{other})$, $T_{User}(e_{other})$, $T_{Client}$, $T_{Client\_p}$ and $T_{Client\_p+1}$ may be time information in a format, which is not intuitively readable by humans, e.g. UNIX time (EPOCH time).

In one embodiment, considering irregularities in the dose event record and/or previous transmissions of at least a part of the dose event record to be received by the receiving unit, all time information which is not in a format that can intuitively be read by a human, may be converted into time information that can intuitively be read by a, human, e.g. the UTC.

In one embodiment, the absolute time of a dose event, $T_{Dose}(e)$, may be determined based on a determination function which converts the dose event related time information $T_{Stamp}(e)$ into a format which is intuitively readable for a human, e.g. the UTC or any other time format suitable to precisely indicate the time of the dose event such that it can be understood by a human without further conversion.

In one embodiment, the absolute time, $T_{Dose}(e)$, of a particular dose event, e, recorded in the dose event record may be determined according to one of the determination functions below,

$$T_{Dose}(e) = g(T_{Client} - (T_{RTC\_trans} - T_{Stamp}(e))),$$

and/or

$$T_{Dose}(e) = j(T_{RTC\_FDE} + T_{Stamp}(e)),$$

and/or

$$T_{Dose}(e) = k(T_{Client\_p+1} - (T_{RTC\_m+1} - T_{Stamp}(e)) * CF),$$

and/or

$$T_{Dose}(e) = l(T_{Client\_p} - (T_{RTC\_m} - T_{Stamp}(e))),$$

In one embodiment, the determination functions $g$, $j$, $k$ and $l$ may be functions of the variables and/or expressions specified in the brackets.

In one embodiment, according to the determination function $g$,

$$T_{Dose}(e) = T_{Client} - (T_{RTC\_trans} - T_{Stamp}(e)).$$

In one embodiment, according to the determination function j:

$$T_{Dose}(e) = T_{RTC\_FDE} + T_{Stamp}(e).$$

In one embodiment, according to the determination function /c:

$$T_{Dose}(e) = T_{Client\_p+1} - (T_{RTC\_m+1} - T_{Stamp}(e)) * CF.$$

In the determination function $k$, $CF$ is a correction factor. The correction factor may be function of time information from the recording unit and of time information from the receiving unit. The correction factor may be configured to compensate time drifts between the time information of the recording unit and the time information of the receiving unit, e.g. between $T_{RTC}$ of the recording unit and the absolute time, e.g. the UTC.

In one embodiment, the correction factor, $CF$ may be determined according to the equation:

$$CF = 1 - \frac{(T_{RTC\_m+1} - T_{RTC\_m}) - (T_{Client\_p+1} - T_{Client\_p})}{(T_{RTC\_m+1} - T_{RTC\_m})}.$$

In one embodiment, according to the determination function $l$:

$$T_{Dose}(e) = T_{Client\_p} - (T_{RTC\_m} - T_{Stamp}(e)).$$

In one embodiment, prior to the determination of $T_{Dose}(e)$, the method may comprise the step of transmitting the at least one part of the dose event record from the recording unit to the receiving unit.

In one embodiment, the transmission may be a wireless transmission, e.g. a transmission via Bluetooth, Bluetooth Low Energy, Near Field Communication (NFC), or any other suitable wireless technology. Alternatively, the transmission may be a wired transmission, e.g. via Universal Serial Bus (USB).

In one embodiment, prior to the transmission of the at least one part of the dose event record, the method may comprise the step of recording information relating to one or more dose events with a recording unit in the dose event record.

Another aspect the present disclosure relates to an apparatus for a medical device. The apparatus comprises a recording unit, an energy source and a storage unit. The recording unit is configured to record information relating to one or more dose events performed with a medical device in a dose event record. The recording unit comprises a timer configured to provide time information for the recording unit, the time information being $T_{RTC}$. The storage unit is configured to store the dose event record. The storage unit may form part of the recording unit. The energy source is configured to provide electrical energy to the recording unit and/or the storage unit.

In one embodiment, the apparatus may be configured to be attachable to the medical device. The medical device may be a medicament delivery device. The medicament delivery device may be a device with which a user can set variable doses, a so-called variable dose device.

The medicament delivery device may be an injection device. The medicament delivery device may be a pen-type device.

The apparatus may be attachable to a dose setting unit, a dose setting mechanism, an activation mechanism, an ejection unit and/or a housing of the medical device.

The apparatus may be configured to be attachable to the medical device, such that a user can perform dose events with the medical device via the apparatus.

The timer of the recording unit may comprise a real time clock. The real time clock may be synchronized with an absolute time, e.g. the UTC, during manufacturing of the recording unit or during assembly of some or all of the components of the apparatus. In detail, the start value of $T_{RTC}$ may be set to correspond to an absolute time when the timer starts to run, e.g. when it is connected to an energy source. Alternatively, the start value of $T_{RTC}$ may be any predefined value, e.g. zero or a value different from zero.

In one embodiment, the value of $T_{RTC}$ at the moment when a first dose event is recorded in the dose event record may be stored as $T_{RTC\_FDE}$. In one embodiment, the value of $T_{RTC}$ at the moment when a first dose event is recorded in the dose event record may not be stored.

In one embodiment, $T_{RTC}$ may be set to zero after at least one part of the dose event record is transmitted to be received by the receiving unit and/or after the transmitted part is received by the receiving unit. In one embodiment, $T_{RTC}$ may be set to zero each time after a part of the dose event record is transmitted to be received by the receiving unit and/or after the transmitted part of the dose event record is received by the receiving unit.

The time information $T_{RTC}$ may be in a format that can be read intuitively by a human such as, e.g. the Coordinated Universal Time, UTC. Alternatively, $T_{RTC}$ may be in a format which is not intuitively readable by a human, e.g. the UNIX time (EPOCH time).

The time information from the recording unit may comprise one of, an arbitrarily selected plurality of, or all of $T_{Stamp}(e)$, $T_{RTC\_trans}$, $T_{RTC\_m}$, $T_{RTC\_m+1}$, and $T_{RTC\_FDE}$, with $T_{stamp}(e)$, $T_{RTC\_trans}$, $T_{RTC\_m}$, $T_{RTC\_m+1}$, and $T_{RTC\_FDE}$ being time information of the recording unit, as described in the foregoing..

The time information from the receiving unit may comprise one of, or all of $T_{Client}$, $T_{Client\_p}$, and $T_{Client\_p+1}$, with $T_{Client}$, $T_{Client\_p}$, and $T_{Client\_p+1}$ being time information of the receiving unit, as described in the foregoing.

In one embodiment the index m may increase by one each time a part of the dose event record is transmitted to be received by the receiving unit. Likewise, the index $p$ may increase by one each time a part of the dose event record is successfully received by the receiving unit.

In one embodiment, the receiving unit may be part of a dose event processing device.

In one embodiment, the value of $T_{RTC}$ at the moment when a first dose event is recorded in the dose event record may be stored in the storage unit as $T_{RTC\_FDE}$. In one embodiment, the value of $T_{RTC}$ at the moment when a first dose event is recorded in the dose event record may not be stored.

In one embodiment, the apparatus is or forms part of an add-on. For example, the apparatus may be an add-on comprising an electronic button. The apparatus may be configured to be attached to or attachable to the medical device. The apparatus may be attachable to a medicament delivery device, e.g. the device as described further above.

Another aspect of the present disclosure relates to a medical device comprising an apparatus, a dose setting unit and a dose ejection unit.

The medical device may be a medicament delivery device, e.g. the device as described further above.

The dose setting unit may be operatively connected to the dose ejection unit, such that a dose which is set with the dose setting unit can be ejected with the dose ejection unit.

The apparatus may be configured to record dose events performed with the medical device in a dose event record. The apparatus may be releasably attached to the medical device. Alternatively, the apparatus may be fixedly attached to the medical device. Alternatively, the apparatus may be integrally formed with the medical device. The apparatus may be an apparatus as described in the foregoing paragraphs.

The medical device may further comprise a holder for holding a medicament container and/or a medicament container with a volume containing medicament. The medicament container may further comprise a stopper configured to move within the volume in order to expel medicament from the medicament container if moved in a distal direction.

The dose ejection unit may comprise a drive unit and a plunger. The drive unit may be configured to provide a force for moving the plunger in an axial direction. The plunger may be configured to transfer the movement driven by the force of the drive unit to the stopper of the medicament container. If moved distally within the volume, the stopper may be configured to expel medicament from the medicament container.

In an embodiment, the plunger may be configured such that a distal end thereof forms the stopper of the medicament container. In other words, the distal end of the plunger may be configured to move within the volume, thereby expelling medicament therefrom, when moved in a distal direction.

In one embodiment, the drive unit may be a mechanism that transfers a user's force to the plunger.

In one embodiment, the drive unit may by a drive spring. The drive spring may be pretensioned or may be tensioned due to a user interaction on the medical device, e.g. during dose setting or selecting.

The medical device may further comprise a cap and/or a needle mount for mounting a needle unit to the device. The cap may be configured to cover a needle fully or partially, when the cap is attached to the medical device and the needle unit is mounted.

Another aspect of the present disclosure relates to a dose event processing device for determining an absolute time, $T_{Dose}(e)$, of one or more recorded dose events, $e_1$ to $e_z$, performed with a medical device, wherein e characterizes one particular dose event of the one or more dose events. The dose event processing device comprises a receiving unit and a processing unit. The receiving unit is configured to receive at least a part of a dose event record containing the one or more recorded dose events $e_1$ to $e_z$. The dose event processing device is configured to determine an absolute time, $T_{Dose}(e)$, of one or more recorded dose events, $e_1$ to $e_z$, recorded in the received part of the dose event record.

In one embodiment, the dose event processing device may be configured to determine the absolute time, $T_{Dose}(e)$, of the one or more recorded dose events $e_1$ to $e_z$ based on a determination function. The determination function for a particular dose event e may be selected depending on an occurrence of an irregularity in the time information of received part of the dose event record, and/or in consideration of the timestamp $T_{Stamp}(e)$ of the particular dose event e with respect to an irregularity in the dose event record, as explained in the forgoing paragraphs.

In one embodiment, the determination function for a particular dose event e may be additionally selected depending on whether a determination period which contains the particular dose event e starts with a reset of $T_{RTC}$.

In one embodiment, the processing unit may be configured to perform the method according to an aspect of the present disclosure.

The dose event processing device may further comprise a user interface. The user interface may be operatively connected to the receiving unit and/or the processing unit. The user interface may be configured to receive a user input, e.g. a user's selection of the determination period in the received part of the dose event record.

The dose event processing device may be or may form part of a mobile device, e.g. a smartphone, a tablet or a portable computer. Alternatively, the dose event processing device may be or may form part of a stationary processing device, e.g. a stationary computer or a server.

Another aspect of the present disclosure relates to a computer program product comprising machine-readable instructions which, when the instructions are executed by a processing unit, causes a dose event processing device to carry out a method for determining an absolute time, $T_{Dose}(e)$, of one or more recorded dose events, $e_1$ to $e_z$, performed with a medical device. the dose event processing device may be a dose event processing device according to an aspect of the present disclosure. The method may be a method according to an aspect of the present disclosure.

Another aspect of the present disclosure relates to a computer-readable storage medium, e.g. a non-transitory computer-readable storage medium, having stored thereon a computer program product comprising machine-readable instructions which, when the instructions are executed by a processing unit, causes a dose event processing device to carry out a method for determining an absolute time, $T_{Dose}(e)$, of one or more recorded dose events, $e_1$ to $e_z$, performed with a medical device. The computer program product may be a computer program product according to an aspect of the present disclosure.

In other words, one aspect of the disclosure relates to a computer-readable storage medium, e.g. a non-transitory computer-readable storage medium, having stored thereon a computer program product comprising machine-readable instructions which, when the instructions are executed by the processing unit of a dose event processing device, causes the dose event processing device to carry out a method for method for determining an absolute time, $T_{Dose}(e)$, of one or more recorded dose events, $e_1$ to $e_z$, performed with a medical device, wherein e characterizes one particular dose event, and wherein the index z is a positive integer.

In one embodiment, the method may be a method according to an aspect of the present disclosure.

In one embodiment, the dose event processing device may be a dose event processing device according to an aspect of the present disclosure.

The method, apparatus and devices according to the present disclosure provide the advantage that an absolute time of one or more dose events performed with a medical device, can be determined precisely and reliably even if there is an interruption in the power supply of the recording unit, which leads to a pause or reset of the timer of the recording unit or if the recording unit is influenced by electromagnetic interferences.

In particular, in contrast to prior art approaches, according to the present disclosure, it is possible to reliably determine $T_{Dose}(e)$ for dose events that were recorded before and after a reset of the timer of the recording unit. This is because in every determination, possible resets or pauses of $T_{RTC}$ are considered and compensated.

Additionally, there is no need for storing the First-Use-Time in the recording unit, which further hardens the method, apparatus and devices against a loss of time information. Due to the use of time information from the recording unit as well as from the receiving unit, a drift of the timer of the recording unit in comparison with an absolute time may be compensated.

It should be noted that all the aspects exemplarily described above can be combined with other aspects or parts thereof as described in the foregoing, even if such a combination of aspects and parts is not explicitly mentioned.

Brief description of the drawings

Figure 1 schematically illustrates steps of a method for determining an absolute time of a recorded dose event performed with a medical device, according to an embodiment of the disclosure.

Figure 2 is a diagram illustrating dose events and corresponding time information of the recording unit and the receiving unit, wherein in the illustrated determination period there is no irregularity in the time information of the dose event record.

Figure 3 is a diagram illustrating dose events and corresponding time information of the recording unit and the receiving unit, wherein in the illustrated determination period there is one irregularity in the time information of the dose event record.

Figure 4 is a diagram illustrating dose events and corresponding time information of the recording unit and the receiving unit, wherein in the illustrated diagram, there are two different determination periods in which there is no irregularity in the time information of the dose event record.

Figure 5 is a diagram illustrating dose events and corresponding time information of the recording unit and the receiving unit, wherein in the illustrated diagram, there are two different determination periods, and the second determination period contains an irregularity in the time information of the dose event record.

Figure 6 is a diagram illustrating dose events and corresponding time information of the recording unit and the receiving unit, wherein in the illustrated diagram, there are two different determination periods, and the second determination period contains two irregularities in the time information of the dose event record.

Figure 7 schematically illustrates an apparatus for a medical device according to an embodiment of the disclosure.

Figure 8 schematically illustrates a dose event processing device according to an embodiment of the disclosure.

**EP 4 350 703 A1**

Figure 9 schematically illustrates a medical device according to an embodiment of the disclosure.

Description of the exemplary embodiments

Identical elements, elements of the same kind and identically or similarly acting elements may be provided with the same reference numerals in the figures.

As outlined before, it is an object of the present disclosure to provide improvements relating to the determination of an absolute time of a recorded dose event, especially if there has been a reset of the real time clock before the dose event was recorded. It is a further object of the present disclosure to remove the need of storing the time of a first dose event performed with a medical device.

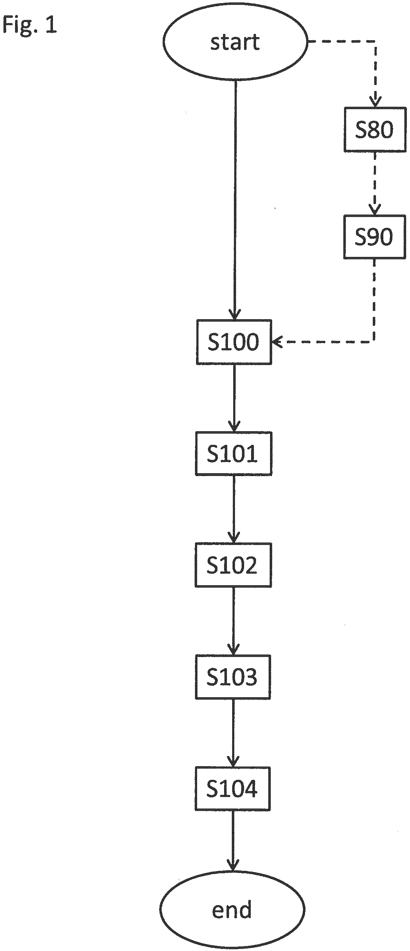

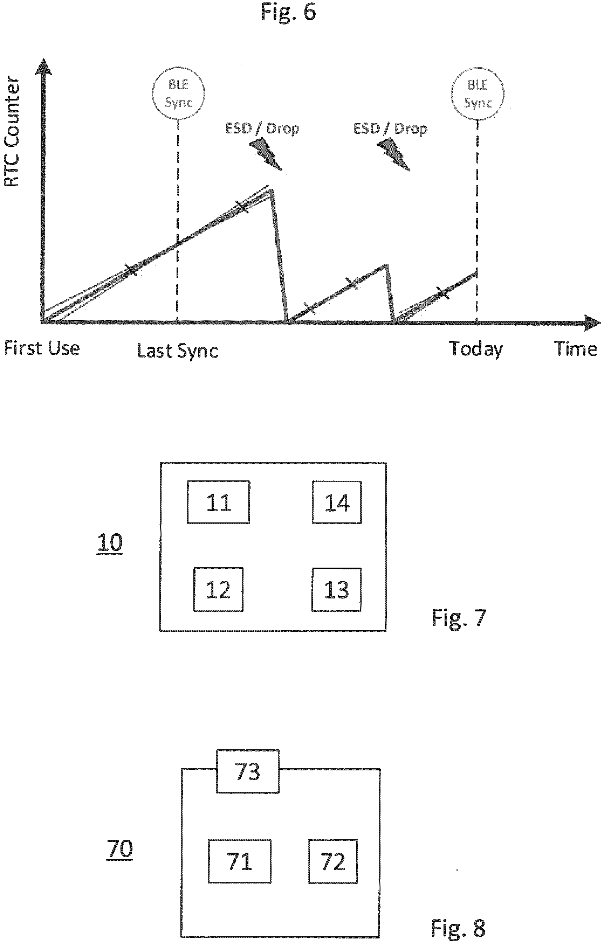

Figure 1 exemplarily illustrates the steps of a method for determining an absolute time, $T_{Dose}(e)$, of one or more recorded dose events, $e_1$ to $e_z$, performed with a medical device, according to an embodiment of the disclosure. In this context, e characterizes one particular dose event of the dose events $e_1$ to $e_z$ recorded in a dose event record. The method starts with the step S100, of receiving at least a part of a dose event record containing at least one dose event e by a receiving unit 71 (see Figure 8).

According to the method, $T_{Dose}(e)$ is determined based on at least one time information from a recording unit 11 (see Figure 7), by which the dose event was recorded for the dose event record, and at least one time information from the receiving unit 71.

The at least one part of the dose event record may comprise information relating to at least one of, an arbitrary selected plurality of, or all of a unique dose event identifier, a dose quantity and a dose event related time information, $T_{stamp}(e)$, of the recording unit 11 which is indicative for the time at which the dose event e was recorded in the at least one part of the dose event record.

The dose event identifier may be a unique number, text or combination of signs, based on which one particular dose event e can be identified unequivocally. The dose quantity may be a quantity or amount of medicament which is set, selected, or output in a single dose event e.

The time information from the recording unit 11 may comprise one of, an arbitrarily selected plurality of, or all of $T_{stamp}(e)$, $T_{RTC\_trans}$, and $T_{RTC\_FDE}$. $T_{Stamp}(e)$ may be dose event related time information of the recording unit 11 which is indicative for the time at which the dose event e was recorded in the dose event record. $T_{RTC\_trans}$ may be a transmission related time information of the recording unit 11 which is indicative for the time at which the at least one part of the dose event record containing the dose event e is transmitted to be received by the receiving unit 71. $T_{RTC\_FDE}$ may be a first dose event time information of the recording unit which is indicative for the time at which a first dose event is recorded by the recording unit 11 in the dose event record.

The time information from the receiving unit 71 may comprise a time, $T_{Client}$, of the receiving unit 71 which is indicative for the time at which the at least one part of the dose event record is received by the receiving unit 71. In one embodiment, the time information of the receiving unit 71 may be synchronized with an absolute time, e.g. the Coordinated Universal Time, UTC.

The recording unit 11 comprises a timer (not illustrated) configured to provide time information for the recording unit 11, the time information being $T_{RTC}$. The timer of the recording unit 11 may comprise a real time clock. The start value of $T_{RTC}$ may be set to correspond to an absolute time at the moment when the timer starts to run, e.g. when the recording unit is connected to an energy source during assembly.

In one embodiment, the value of $T_{RTC}$ at the moment when a first dose event is recorded in the dose event record may be stored as $T_{RTC\_FDE}$.

In one embodiment, the value of $T_{RTC}$ is set to be zero when a first dose event is recorded in the dose event record. In one embodiment, $T_{RTC}$ may be set to zero after at least one part of the dose event record is transmitted to be received by the receiving unit 71 and/or after the transmitted part is received by the receiving unit 71. In one embodiment, $T_{RTC}$ may be set to zero each time after a part of the dose event record is transmitted to be received by the receiving unit and/or after the transmitted part of the dose event record is received by the receiving unit 71.

After the at least one part of the dose event record is received by the receiving unit 71, in step S101, a determination period in the received part of the dose event record is defined.

The determination period is a portion of the received part of the dose event record, which contains all dose events, $e_i$ to $e_k$, for which for which an absolute time $T_{Dose}(e)$ shall be determined. For example, the determination period may be defined according to unique dose event identifiers of dose events in the received part of the dose event record for which $T_{Dose}(e)$ has not been determined yet. Alternatively, the determination period may be selected by a user, e.g. via a user interface 73 (see Figure 8) operatively connected to the receiving unit 71. Alternatively, the determination period may extend over the whole length of the received part of the dose event record.

The dose event identifier may be a unique number, text or combination of signs, based on which one particular dose event can be identified unequivocally. Apart from the unique dose event identifier, the dose record comprises information on a dose quantity for each dose and a dose event related time information, $T_{stamp}(e)$, of the recording unit

11

which is indicative for the time at which the dose event e was recorded in the dose event record. The dose quantity may be a quantity or amount of medicament which is set, selected, or output in a single dose event.

According to step S102, the time information contained in the dose event record within the determination period is evaluated with respect to an occurrence of an irregularity.

The evaluation may be based on time information from the recording unit 11 and/or based on information stored in the dose event record and/or based on time information from the receiving unit 71.

In one embodiment, the occurrence of an irregularity is evaluated based on a detection of a change, e.g. a step change, in a variable $\Delta$, which is indicative for a time deviation between the recording unit 11 and the receiving unit 71. After at least one part of the dose event record is received by the receiving unit 71, $\Delta$ may be determined based on time information from the recording unit 11 and time information from the receiving unit 71, e.g. $T_{RTC\_m}$ and $T_{Client\_p}$. In one embodiment, $T_{RTC\_m}$ is a transmission related time information of the recording unit which is indicative for the time, m, at which the at least one part of the dose event record is transmitted to be received by the receiving unit, and $T_{Client\_p}$ is a time information from the receiving unit which is indicative for the time, p, at which the at least one part of the dose event record is received by the receiving unit. In one embodiment, $\Delta$ may be determined according to the equation: $\Delta = T_{Client\_p} - T_{RTC\_m}$.

In one embodiment, $\Delta$ is determined each time a part of the dose event record is received by the receiving unit 71. The values for each determined $\Delta$ are stored in a memory unit (not shown) as $\Delta_{pre\_i}$, for further processing at a later point in time. Alternatively, $\Delta$ may be determined less often, e.g. only every second, third fourth, fifth, tenth, twentieth or fiftieth time at which a part of the dose event record is received by the receiving unit. In one embodiment, only the most recent determined $\Delta$ may be stored in the recording unit 11 as $\Delta_{pre}$.

In one embodiment, the change may be detected by comparing the most recent value of $\Delta$ with one of the stored $\Delta_{pre\_i}$ or the stored $\Delta_{pre}$.

The irregularity may be a reset event of the recording unit 11 at which the timer of the recording unit 11 is reset resulting in a change of $T_{RTC}$ to a default value of $T_{RTC}$. In one embodiment, the default value is zero. Alternatively, the default value may be different from zero.

In step S103, a determination function for determining $T_{Dose}(e)$ for each dose event $e_i$ to $e_k$ is selected, based on the result of the evaluation regarding irregularities in the time information of the dose event record. Further, the determination function is selected in consideration of the timestamp $T_{Stamp}(e)$ of a dose event e with respect to an irregularity in the dose event record. In one embodiment, the determination function may be selected in additional consideration of a value of $T_{RTC}$ at the start of the determination period.

In step S104, the absolute time, $T_{Dose}(e)$, for each dose event, $e_i$ to $e_k$, within the determination period is determined, based on the determination function selected in step S103.

In one embodiment, prior to the determination of $T_{Dose}(e)$, the method may comprise the step S90, i.e. transmitting the at least one part of the dose event record from the recording unit 11 to the receiving unit 71 In one embodiment, prior to the transmission S90, the method may comprise the step S80, i.e. recording information relating to one or more dose events, $e_1$ to $e_z$, performed with a medical device with a recording unit 71 in the dose event record.

The determination function $f$ may be a function of the time information from the recording unit and the time information from the receiving unit.

In one embodiment, the determination function $f$ may be such that $T_{Dose}(e) = f(T_{Client}, T_{RTC\_trans}, T_{Stamp}(e))$.

In one embodiment, the determination function $f$ may be a function of the time information from the receiving unit and a difference of different time information from the recording unit, e.g. $T_{RTC\_trans} - T_{stamp}(e)$, such that $T_{Dose}(e) = f(T_{Client}, (T_{RTC\_trans} - T_{Stamp}(e)))$.

In one embodiment, the determination function $f$ may be a function of a difference between the time information from the receiving unit and the time information from the recording unit, such that $T_{Dose}(e) = f(T_{Client} - (T_{RTC\_trans} - T_{Stamp}(e)))$.

In one embodiment, the determination function f may be such that $T_{Dose}(e) = T_{Client} - (T_{RTC\_trans} - T_{Stamp}(e))$.

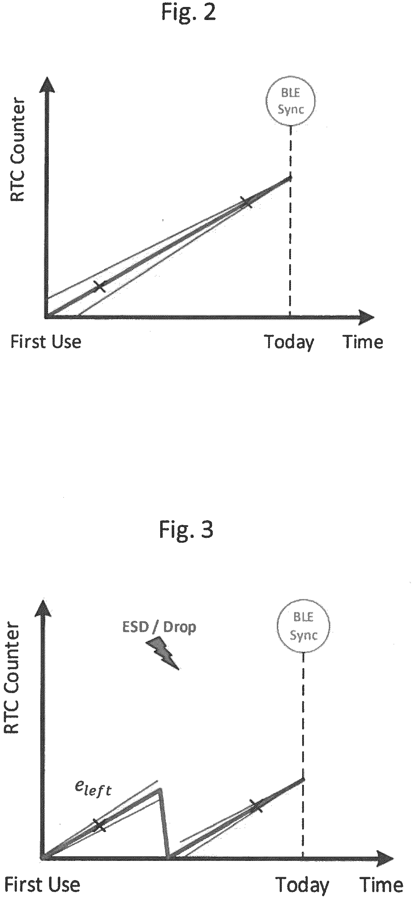

In one embodiment, a determination function, $g$, may be selected, S103, if the determination period does not contain any irregularity. This is exemplarily illustrated by the curve of Figure 2. The x-axis of the diagram represents the absolute time with which the receiving unit is synchronized, e.g. the UTC. The y-axis of the diagram represents the value of $T_{RTC}$. The vertical dotted line indicates $T_{Client}/T_{Client\_p}$ of the receiving unit 71, when the at least one part of the dose event record is received by the receiving unit 71, indicated by the label "BLE Sync". The crosses on the curve represent the dose events $e_i$ to $e_k$ for which $T_{Dose}(e)$ shall be determined. In Figure 2, the part of the dose event record starting with the "First Use" until "today" may be transmitted to be received by the receiving unit. The determination period may extend between "First Use" and "today".

In one embodiment, $g$ may also be selected, S103, if the dose event for which $T_{Dose}(e)$ is to be determined was recorded after a last reset of $T_{RTC}$ in the determination period. In this context, the last reset of $T_{RTC}$ is a reset of $T_{RTC}$ in the determination period after which there is no subsequent reset of $T_{RTC}$ in the determination period. In other words, if there is no reset event between the last reset of $T_{RTC}$ and the point in time at which the at least one part of the dose event record is transmitted to be received by the receiving unit 71.

Referring to Figure 3, this is the right part of the illustrated curve after the reset of $T_{RTC}$ (i.e. when $T_{RTC}$ = 0), which is indicated by the caption "ESD/Drop", until the vertical dotted line, i.e. when a first part of the dose event record is received by the receiving unit 71, as indicated by the caption "BLE Sync". In Figure 3, the determination period extends from "First Use" until "today".

For the sake of referencing to certain parts of the curve, the determination period can be divided in two portions, one being the portion before the reset of $T_{RTC}$, i.e. the left part of the curve and the other being the portion after the reset of $T_{RTC}$, i.e. the right part of the curve.

As "BLE Sync" is the first transmission of the dose event record, it is evident that there is no $\Delta_{pre}$ stored which could be used for the determination of a change in the variable $\Delta$ and the determination of a reset of $T_{RTC}$. In this scenario, the determination that there is a reset of $T_{RTC}$ in the determination period may be made based on information stored in the dose event record, e.g. $T_{Stamp}(e)$ in combination with the dose event identifier. If $T_{Stamp}(e)$ for subsequent dose events does not increase, this indicates a reset of $T_{RTC}$ between these dose events.

For all dose events of the second portion, i.e. the right part of the curve, the determination function $g$ is selected.

The determination function $g$ is a function of the time information from the recording unit 11 and the time information from the receiving unit 71, such that $T_{Dose}(e) = g(T_{Client}, T_{RTC\_trans}, T_{Stamp}(e))$. More particularly, the determination function $g$ may be a function of the time information from the receiving unit 71 and a difference of time information from the recording unit 11, e.g. $T_{RTC\_trans} - T_{stamp}(e)$, such that $T_{Dose}(e) = g(T_{Client}, (T_{RTC\_trans} - T_{Stamp}(e)))$. Even more particularly, the determination function $g$ may be a function of a difference between the time information from the receiving unit 71 and the time information from the recording unit 11, e.g. $T_{RTC\_trans} - T_{Stamp}(e)$, such that $T_{Dose}(e) = g(T_{Client} - (T_{RTC\_trans} - T_{Stamp}(e)))$. **In** one embodiment, the determination function $g$ is such that:

$$T_{Dose}(e) = T_{Client} - (T_{RTC\_trans} - T_{Stamp}(e)).$$

Regarding the dose event $e_{left}$ illustrated by the cross on the left part of the curve in Figure 3, i.e. the first portion of the determination period, the conditions for selecting the determination function $g$, are not fulfilled because there is a reset of $T_{RTC}$ between $e_{left}$ and the end of the determination period. In other words, the absolute time $T_{Dose}(e_{left})$ of this dose event $e_{left}$ cannot be reliably determined with the determination function $g$.

However, in one embodiment, for the dose event $e_{left}$ and all other recorded dose events in the first portion, a determination function, $i$, may be selected ..

The determination function $i$ is a function of $T_{stamp}(e)$, $T_{User}(e_{other})$ and $T_{Stamp}(e_{other})$. As described above, $T_{Stamp}(e)$ is the dose event related time information of the recording unit which is indicative for the time at which the dose event e for which the absolute time is to be determined, was recorded in the dose event record. $T_{User}(e_{other})$ is a user entered time of one other dose event, $e_{other}$, which was recorded in the same portion of the determination period as the dose event $e$, for which $T_{Dose}(e)$ is to be determined. In this context, "same portion" means that there is no irregularity or reset of $T_{RTC}$ in the dose event record between the dose event e and the dose event $e_{other}$ (cf. e.g. the two dose events in Figure 6 between the two "ESD/Drop" labels). $T_{Stamp}(e_{other})$ is a dose event related time information of the recording unit 11 which is indicative for the time at which this other dose event, $e_{other}$, was recorded in the dose event record.

In one embodiment, the determination function $i$ is a function of $T_{User}(e_{other})$ and a difference between the dose event related time information of the dose events e and $e_{other}$ such that:

$$T_{Dose}(e) = T_{User}(e_{other}) - T_{Stamp}(e_{other}) + T_{Stamp}(e)$$

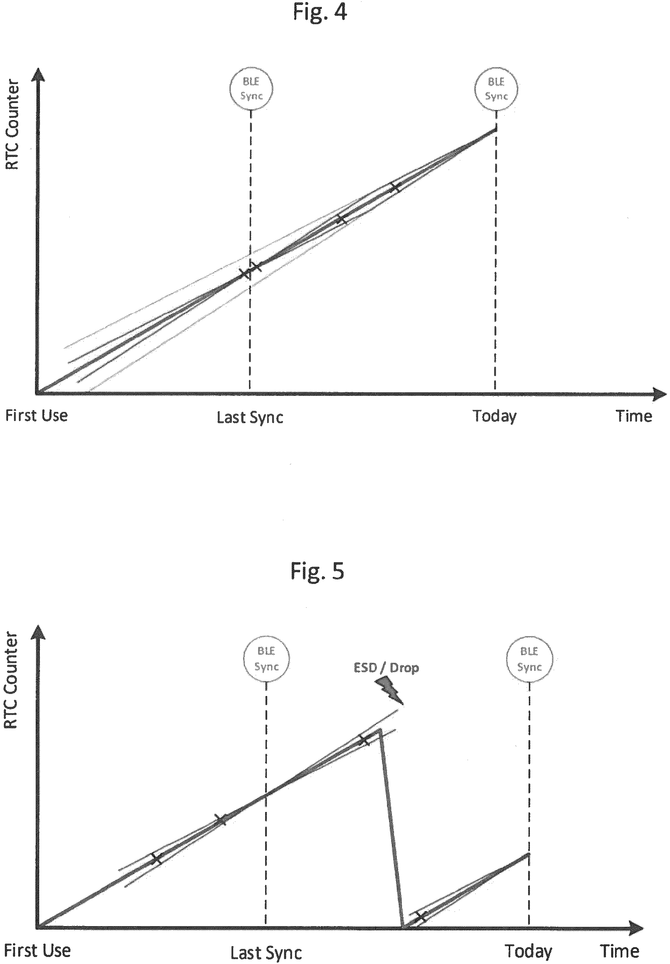

In one embodiment as illustrated in Figure 5, the determination period extends between an earlier point in time, m, at which at least one part of the dose event record is transmitted to be received by the receiving unit 71 and a later point in time, $m + 1$, at which another part of the dose event record is transmitted to be received by the receiving unit 71. The corresponding time information of the receiving unit 71 at which these parts of the dose event record are received is given by the indices $p$ and $p + 1$, respectively. In detail, $T_{Client\_p}$ is a time of the receiving unit 71 which is indicative for the time at which the at least one part of the dose event record is received by the receiving unit 71 (cf. the left "BLE Sync" label in Figure 5). Correspondingly, $T_{Client\_p+1}$ is a time of the receiving unit 71 which is indicative for the time at which the other part of the dose event record is received by the receiving unit 71 (cf. the right "BLE Sync" label in Figure 5).

The determination period illustrated in Figure 5 contains one reset of $T_{RTC}$. Further, in the illustrated curve, $T_{RTC}$ is not at its default value, e.g. zero, at the start of the determination period. However, for the determination of

$T_{Dose}(e)$, $T_{RTC}$ may also be at its default value at the start of the determination period.

As regards dose events recorded after the reset of $T_{RTC}$ indicated by the label "ESD/Drop", the conditions for selecting the determination function $g$ are fulfilled. As regards dose events recorded before the reset of $T_{RTC}$ the conditions for selecting the determination function $g$ are not fulfilled. For these dose events, a determination function, $h$, is selected.

In other words, the determination function $h$ is selected if the determination period starts after a point in time, $p$, at which at least a part of the dose event record had been received by the receiving unit 71 and if the dose event, $e$, for which $T_{Dose}(e)$ is to be determined was recorded before a first reset of $T_{RTC}$ in the determination period. If these conditions are fulfilled, the determination function $h$ may be selected even if $T_{RTC}$ is not at its default value at the start of the determination period.

In one embodiment, the determination function $h$ is a function of $\Delta_{pre}$ and $T_{Stamp}(e)$), wherein $\Delta_{pre}$ is determined based on time information from the recoding unit, e.g. $T_{RTC\_m}$ and time information from the receiving unit, e.g. $T_{Client\_p}$. Referring to the curve illustrated in Figure 5, $\Delta_{pre}$ may be determined according to: $\Delta_{pre} = T_{Client\_p} - T_{RTC\_m}$.

In one embodiment, the determination function $h$ may correspond to a determination function $l$ which is described below. The determination function $h$ (or $l$) may be such that:

$$T_{Dose}(e) = \Delta_{pre} + T_{Stamp}(e) = T_{Client\_p} - T_{RTC\_m} + T_{Stamp}(e).$$

Referring to Figure 6, the difference to Figure 5 is that the illustrated curve contains two resets of $T_{RTC}$. In other words, there are two resets of $T_{RTC}$ in the determination period. As described before, regarding all dose events recorded before the first reset of $T_{RTC}$ in the determination period, the determination function $h$ is selected. For all dose events recorded after the last reset of $T_{RTC}$ in the determination period, the determination function $g$ is selected.

In one embodiment, the above-mentioned determination function $i$ is selected, if the dose event, $e$, for which $T_{Dose}(e)$ is to be determined was recorded between an $n$-th reset of $T_{RTC}$ and an $(n + 1)$-th reset of $T_{RTC}$ in the determination period. In the illustrated example, $n = 1$. The dose events for which $T_{Dose}(e)$ is determined with determination function $i$ are illustrated in Figure 6 by the two crosses between the left "ESD/Drop" and the right "ESD/Drop" label. However, in one embodiment, the determination function $i$ may be selected for all $n \geq 1$. According to the determination function $i$:

$$T_{Dose}(e) = .T_{User}(e_{other}) - T_{Stamp}(e_{other}) + T_{Stamp}(e)$$

In one embodiment, the determination function $i$ may also be selected for all dose events which do not fulfill the conditions for selecting the determination function $g$ or the determination function $h$. For example, this includes dose events recorded before a first irregularity in the determination period, if the determination period starts with a first dose event recorded in the dose event record (cf. the dose event "$e_{left}$" in Figure 3).

In one embodiment, the determination function, $j$, may be selected if the dose event for which $T_{Dose}(e)$ is to be determined was recorded between a first dose event recorded in the dose event record and a first irregularity in the determination period, e.g. the dose event "$e_{left}$" in Figure 3. In one embodiment, according to the determination function j:

$$T_{Dose}(e) = T_{RTC\_FDE} + T_{Stamp}(e).$$

As described above in the summary, $T_{RTC\_FDE}$ may be a first dose event time information of the recording unit which is indicative for the time at which a first dose event recorded by the recording unit 11 in the dose event record. As outlined in the summary, the initial value of $T_{RTC}$ may be to set according to an absolute time, e.g. the Coordinated Universal Time, UTC, during manufacturing of the recording unit. That is, the initial value of $T_{RTC}$ may be set to correspond to an absolute time when the timer starts to run, e.g. at the moment when the recording unit is connected to an energy source during assembly. Consequently, $T_{RTC\_FDE}$ may correspond to the value of $T_{RTC}$ at the moment when a first dose event is recorded in the dose event record.

. In one embodiment illustrated in Figure 4, a determination function, $k$, may be selected, if there is no reset of $T_{RTC}$ in the determination period. Especially if $T_{RTC}$ is not at its default value at the start of the determination period, the determination function $k$ may be selected. This is illustrated by the dose events between the two "BLE Sync" labels in Figure 4.

In one embodiment, according to the determination function $k$:

$$T_{Dose}(e) = T_{Client\_p+1} - \left(T_{RTC\_m+1} - T_{Stamp}(e)\right) * CF$$

In the determination function *k*, *CF* is a correction factor. The correction factor is a function of time information from the recording unit and of time information from the receiving unit. In one embodiment, the correction factor, *CF* may be determined according to the equation:

$$CF = 1 - \frac{\left(T_{RTC\_m+1} - T_{RTC\_m}\right) - \left(T_{Client\_p+1} - T_{Client\_p}\right)}{\left(T_{RTC\_m+1} - T_{RTC\_m}\right)}.$$

As described above, $T_{RTC\_m}$ is a transmission related time information of the recording unit which is indicative for a first point in time, *m*, at which at least a first part of the dose event record had been transmitted to be received by the receiving unit. $T_{RTC\_m+1}$ is a transmission related time information of the recording unit which is indicative for a second point in time, *m* + 1, at which at least a second part of the dose event record is transmitted to be received by the receiving unit. $T_{Client\_p}$ is a time information of the receiving unit which is indicative for a third point time, *p*, at which the first part of the dose event record had been received by the receiving unit (cf. the left "BLE Sync" label in Figure 4). In one embodiment, the third point in time may be earlier than the second point in time, which is not illustrated but will be slightly before the right "BLE Sync" label in Figure 4. $T_{Client\_p+1}$ is a time information of the receiving unit which is indicative for a fourth point in time, *p* + 1, at which the second part of the dose event record is received by the receiving unit (cf. the right "BLE Sync" label in Figure 4).

Figure 7 schematically illustrates an apparatus 10 for a medical device according to embodiment of the present disclosure. The apparatus 10 comprises a recording unit 11, a storage unit 12, an energy source 13 and a transmission unit 14. The recording unit is configured to record information relating to one or more dose events performed with a medical device in a dose event record.

The recording unit 11 comprises a timer configured to provide time information for the recording unit, the time information being $T_{RTC}$. The storage unit 12 is configured to store the dose event record. The storage unit 12 may form part of the recording unit. The energy source 13 is configured to provide electrical energy to the recording unit 11 and/or the storage unit 12 and/or the transmission unit 14.

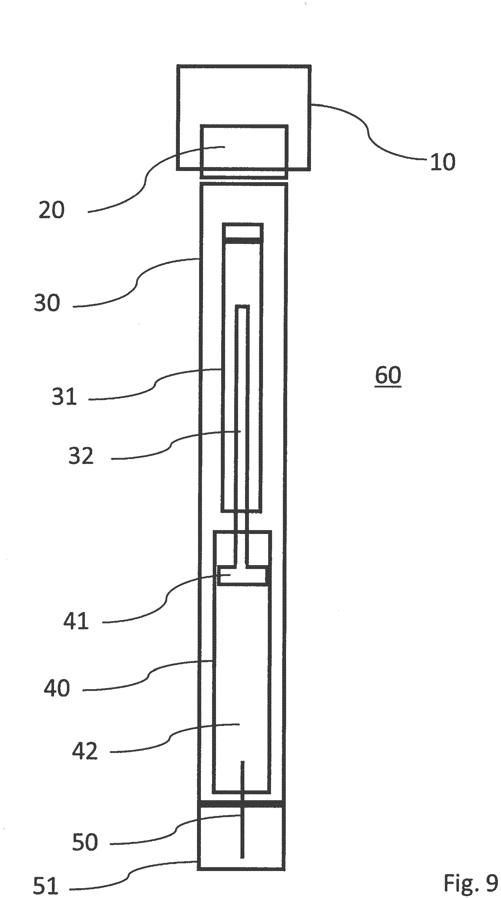

In one embodiment, the apparatus 10 is configured to be attachable to the medical device 60, e.g. a medicament delivery device as illustrated in Figure 9. As shown, the apparatus 10 is configured to be attachable to a dose setting unit 20 of the medical device 60, such that a user can perform dose events with the medical device 60 via the apparatus 10.

In one embodiment, the apparatus 10 may be attachable to one or more of a dose setting unit, an activation mechanism, an ejection unit and/or the housing of a medical device.

As shown in Figures 2 to 6, in one embodiment, the value of $T_{RTC}$ may be set to a default value, i.e. zero, when a first dose event is recorded in the dose event record. Alternatively, the default value of $T_{RTC}$ may be different from zero, as described in the summary above.

The transmission unit 14 is configured to transmit the at least one part of the dose event record and a transmission related time information, $T_{RTC\_trans}$, to a receiving unit 71. The transmission related time information is indicative for the time at which the at least one part of the dose event record containing the dose event e is transmitted to be received by the receiving unit 71. The receiving unit 71 may be part of a dose event processing device 70 (see Figure 8).

Figure 8 schematically illustrates a dose event processing device 70, according to embodiment of the disclosure. The dose event processing device is configured to determine an absolute time, $T_{Dose}(e)$, of one or more recorded dose events, $e_1$ to $e_z$, performed with a medical device, wherein e characterizes one particular dose event of the one or more dose events $e_1$ to $e_z$. The dose events are recorded in a dose event record.

The dose event processing device 70 comprises a receiving unit 71 configured to receive at least a part of a dose event record containing the one or more recorded dose events $e_1$ to $e_z$, and a processing unit 72. The dose event processing device 70, more specifically the processing unit 72 thereof, is configured to determine an absolute time, $T_{Dose}(e)$, of the one or more recorded dose events $e_1$ to $e_z$ recorded in the received part of the dose event record.

In one embodiment, the dose event processing device 70, more specifically the processing unit 72 thereof, is configured to perform the method according to one of the foregoing paragraphs and/or the summary above.

The dose event processing device 70 further comprises a user interface 73 operatively connected to the receiving unit 71 and the processing unit 72. The user interface 73 is configured to receive a user input. Among others, the user input may be a selection of a determination period in the received part of the dose event record.

In one embodiment, the dose event processing device 70 may be a mobile device, e.g. a smartphone.

The dose event processing device 70 is configured to determine the absolute time, $T_{Dose}(e)$, of the one or more recorded dose events $e_1$ to $e_z$ based on a determination function. The determination function for a particular dose event e is selected depending on an occurrence of an irregularity in the time information of received part of the dose event record, e.g. a reset of $T_{RTC}$ and in consideration of the timestamp $T_{Stamp}(e)$ of the particular dose event e with respect to the irregularity in the dose event record, as explained in the foregoing paragraphs and the summary above.

In one embodiment, a determination function, $g$, is selected if the determination period does not contain any irregularity or if the dose event e for which $T_{Dose}(e)$ is to be determined was recorded after a last reset of $T_{RTC}$ in the determination period, wherein the last reset of $T_{RTC}$ is a reset of $T_{RTC}$ in the determination period after which there is no subsequent reset of $T_{RTC}$ in the determination period.

The occurrence of an irregularity is evaluated based on a detection of a change in a variable $\Delta$ as explained in the foregoing paragraphs and the summary above. Alternatively, or additionally, the occurrence of an irregularity may be evaluated based on the values of $T_{Stamp}(e)$ of subsequent dose events as explained in the foregoing paragraphs and the summary above.

Alternatively, or additionally, a determination function, $h$, is selected, if the determination period contains one or more resets of $T_{RTC}$, if the determination period starts after a point in time, p, at which at least a part of the dose event record had been received by the receiving unit 71 and if the dose event for which $T_{Dose}(e)$ is to be determined was recorded before a first reset of $T_{RTC}$ in the determination period.

Alternatively, or additionally, a determination function, $i$, is selected, if neither the conditions for selecting $g$ or $h$, are fulfilled. For example, $i$ may be selected if the dose event e for which $T_{Dose}(e)$ is to be determined was recorded between an $n$-th reset of $T_{RTC}$ and an $(n + 1)$-th reset of $T_{RTC}$ in the determination period, with $n \geq 1$.

In one embodiment, the determination functions may be such that:

$$T_{Dose}(e) = g(T_{Client} - (T_{RTC\_trans} - T_{Stamp}(e))),$$

and/or

$$T_{Dose}(e) = h(\Delta_{pre} + T_{Stamp}(e)),$$

and/or

$$T_{Dose}(e) = i(T_{User}(e_{other}) - T_{Stamp}(e_{other}) + T_{Stamp}(e)),$$

wherein

a) $T_{Stamp}(e)$ is a dose event related time information of the recording unit 11 which is indicative for the time at which the dose event e was recorded in the dose event record;

b) $T_{RTC\_trans}$ is a transmission related time information of the recording unit 11 which is indicative for the time at which the at least one part of the dose event record is transmitted to be received by the receiving unit 71;

c) $T_{RTC\_m}$ is a transmission related time information of the recording unit 11 which is indicative for the time, $m$, at which the at least one part of the dose event record is transmitted to be received by the receiving unit 71;

d) $T_{Client}$ is a time information from the receiving unit 71 which is indicative for the time at which the at least one part of the dose event record is received by the receiving unit 71;

e) $T_{Client\_p}$ is a time information from the receiving unit 71 which is indicative for the time, $p$, at which the at least one part of the dose event record is received by the receiving unit 71;

f) $\Delta_{pre}$ is a variable indicative for a time deviation between the recording unit 11 and the receiving unit 71 which is determined based on time information from the recording unit 11 and time information from the receiving unit 71, e.g. based on $T_{RTC\_m}$ and $T_{Client\_p}$,

g) $T_{User}(e_{other})$ is a user entered time of one other dose event $e_{other}$ which was recorded in the dose event record in a same portion of the determination period as the dose event e;

h) $T_{Stamp}(e_{other})$ is a dose event related time information of the recording unit 11 which is indicative for the time at which the other dose event $e_{other}$ was recorded in the dose event record; and

In one embodiment, $g$, $h$ and $i$ are functions of the variables and/or expressions specified in the brackets.
In one embodiment, the determination functions may be such that:

$$T_{Dose}(e) = g(T_{Client} - (T_{RTC\_trans} - T_{Stamp}(e))),$$

and/or

$$T_{Dose}(e) = j(T_{RTC\_FDE} + T_{Stamp}(e)),$$

and/or

$$T_{Dose}(e) = k(T_{Client\_p+1} - (T_{RTC\_m+1} - T_{Stamp}(e)) * CF),$$

and/or

$$T_{Dose}(e) = l(T_{Client\_p} - (T_{RTC\_m} - T_{Stamp}(e))),$$

wherein

a) $T_{Stamp}(e)$ is a dose event related time information of the recording unit 11 which is indicative for the time at which the dose event e was recorded in the dose event record;

b) $T_{RTC\_trans}$ is a transmission related time information of the recording unit 11 which is indicative for the time at which the at least one part of the dose event record is transmitted to be received by the receiving unit 71;

c) $T_{RTC\_m}$ is a transmission related time information of the recording unit 11 which is indicative for a first point in time, m, at which the at least one part of the dose event record is transmitted to be received by the receiving unit 71;

d) $T_{RTC\_FDE}$ is a first dose event time information of the recording unit 11 which is indicative for the time at which a first dose event is recorded in the dose event record;

e) $T_{RTC\_m+1}$ is a transmission related time information of the recording unit 11 which is indicative for a second point in time, $m + 1$, at which at least a second part of the dose event record is transmitted to be received by the receiving unit 71, the second point in time, $m + 1$, being a point in time after the first point in time, $m$, at which a first part of the dose event record had been transmitted to be received by the receiving unit;

f) $T_{Client}$ is a time information from the receiving unit 71 which is indicative for the time at which the at least one part of the dose event record is received by the receiving unit 71;

g) $T_{Client\_p}$ is a time information from the receiving unit 71 which is indicative for a third point in time, $p$, at which the at least one part of the dose event record is received by the receiving unit 71;

h) $T_{Client\_p+1}$ is a time information of the receiving unit 71 which is indicative for a fourth point in time, $p + 1$, at which the second part of the dose event record is received by the receiving unit 71, the fourth point in time, $p + 1$, being a point in time after a third point in time, $p$, at which the first part of the dose event record had been received by the receiving unit;

i) $CF$ is a correction factor, which is a function of time information from the recording unit 11 and of time information from the receiving unit 71, e.g.

$$CF = 1 - \frac{(T_{RTC\_m+1} - T_{RTC\_m}) - (T_{Client\_p+1} - T_{Client\_p})}{(T_{RTC\_m+1} - T_{RTC\_m})}.$$

Figure 9 schematically illustrates a medical device 60 according to an embodiment of the disclosure. The illustrated medical device 60 is a medicament delivery device comprising an apparatus 10, a dose setting unit 20 and a dose ejection unit. The dose ejection unit is contained in a housing 30. The apparatus 10 is releasably attached to the dose setting unit 20 of the medicament delivery device.

The dose setting unit 20 is configured to set a dose to be ejected in a dose setting operation. The dose setting unit 20 is operatively connected to the dose ejection unit, such that the set dose can be ejected from the medicament delivery device in a dose ejection operation.

The apparatus 10 is configured to record dose events performed with the medical device in a dose event record.

The apparatus 10 may be an apparatus as described in the foregoing paragraphs and/or in the summary above.

The dose ejection unit comprises a drive unit 31 and a plunger 32.

The medicament delivery device further comprises a medicament container 40 with a volume 42 containing the medicament and a stopper 41 movable within the volume 42 in order to discharge medicament from the medicament container if moved in a distal direction.

The drive unit 31 is configured to provide a force for moving the plunger 32 in an axial direction. The plunger 32 is configured to transfer the movement driven by the force of the drive unit 31 to the stopper 41 of the medicament container 40, thereby moving the stopper 41 within respect to the volume 42.

In an embodiment, the plunger 32 may be configured such that a distal end thereof forms the stopper of the medicament container. In other words, the distal end of the plunger 32 may be configured to move within the volume, thereby discharging medicament therefrom, when moved in a distal direction.

In one embodiment, the drive unit may be a mechanism that transfers a drive energy from a user to the plunger.

In one embodiment, the drive unit may by a drive spring. The drive spring may be pretensioned or may be tensioned due to a user interaction on the medical device.

As illustrated in Figure 9, the medical device 60 may further comprise a needle 50 and a cap 51. The cap 51 may be configured to cover the needle 50 fully or partially, when the cap 51 is attached to the medical device.

In one embodiment, the medical device may be a medicament delivery device. In one embodiment the medicament delivery device may be a device with which a user can set variable doses, a so-called variable dose device. In one embodiment, the medicament delivery device may be an injection device: In one embodiment, the medicament delivery device may be a pen-type device.

One embodiment of the disclosure, which is not illustrated in the Figures, relates to a computer program product comprising machine-readable instructions which, when the instructions are executed by a processing unit, causes a dose event processing device to carry out a method for determining an absolute time, $T_{Dose}(e)$, of one or more recorded dose events, $e_1$ to $e_z$, performed with a medical device. The dose event processing device may be a dose event processing device according to an embodiment or aspect of the present disclosure. The method may be a method according to an embodiment or aspect of the present disclosure.

One embodiment of the disclosure, which is not illustrated in the Figures, relates to a computer-readable storage medium, e.g. a non-transitory computer-readable storage medium, having stored thereon a computer program product comprising machine-readable instructions which, when the instructions are executed by a processing unit, causes a dose event processing device to carry out a method for determining an absolute time, $T_{Dose}(e)$, of one or more recorded dose events, $e_1$ to $e_z$, performed with a medical device. The computer program product may be a computer program product according to an embodiment or aspect of the present disclosure.

One embodiment of the disclosure, which is not illustrated in the Figures, relates to a computer-readable storage medium, e.g. a non-transitory computer-readable storage medium, having stored thereon a computer program product comprising machine-readable instructions which, when the instructions are executed by the processing unit of a dose event processing device, causes the dose event processing device to carry out a method for method for determining an absolute time, $T_{Dose}(e)$, of one or more recorded dose events, $e_1$ to $e_z$, performed with a medical device, wherein e characterizes one particular dose event, and wherein the index z is a positive integer.

In one embodiment, the method may be a method according to an embodiment or aspect of the present disclosure.

In one embodiment, the dose event processing device may be a dose event processing device according to an embodiment or aspect of the present disclosure.

As mentioned in the summary, the terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th'edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide. Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir®); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba®); B29-N-(N-lithocholyl-gamma-glutamyl)-des(B30) human insulin; B29-N-($\omega$-carboxyheptadecanoyl)-des(B30) human insulin and B29-N-($\omega$-carboxyheptadecanoyl) human insulin.

Examples of GLP-1, GLP-1 analogues and GLP-1 receptor agonists are, for example, Lixisenatide (Lyxumia®), Exenatide (Exendin-4, Byetta®, Bydureon®, a 39 amino acid peptide which is produced by the salivary glands of the Gila monster), Liraglutide (Victoza®), Semaglutide, Taspoglutide, Albiglutide (Syncria®), Dulaglutide (Trulicity®), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro®), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc®), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

It should be understood that all features and parts as described with respect to the aspects in the summary can be used for the embodiments described in this section, even if not all of those combinations are explicitly mentioned.

List of reference signs

10 apparatus
11 recording unit
12 storage unit
13 energy source
14 transmission unit
20 dose setting unit
30 housing
31 drive unit
32 plunger
40 medicament container
41 stopper
42 volume
50 needle
51 cap
60 medical device
70 dose event processing device
71 receiving unit
72 processing unit
73 user interface

S80 recording dose events in a dose event record
S90 transmitting the dose event record to a receiving unit
S100 receiving the dose event record
S101 defining a determination period
S102 evaluating the time information of the dose event record in the determination period
S103 selecting a determination function
S104 determining the absolute time of a recorded dose event

**Claims**

1. Method for determining an absolute time, $T_{Dose}(e)$, of one or more recorded dose events ($e_1$ to $e_z$) performed with a medical device, wherein e characterizes one particular dose event; wherein the one or more dose events are recorded in a dose event record;

   wherein at least one part of the dose event record is received (S100) by or receivable by a receiving unit (71); and wherein $T_{Dose}(e)$ is determined (S104) based on at least one time information from a recording unit (11), by which the dose event was recorded for the dose event record, and at least one time information from the receiving unit (71).

2. The method of claim 1, wherein

   a) the time information from the recording unit (11) comprises one of, an arbitrarily selected plurality of, or all of:

      a dose event related time information, $T_{stamp}(e)$, of the recording unit (11) which is indicative for the time at which the dose event (e) was recorded in the dose event record; and
      a transmission related time information, $T_{RTC\_trans}$, of the recording unit (11) which is indicative for the time at which the least one part of the dose event record is transmitted to be received by the receiving unit (71), and/or wherein

b) the time information from the receiving unit (71) comprises a time, $T_{Client}$, of the receiving unit (71) which is indicative for the time at which the at least one part of the dose event record is received by the receiving unit (71).

3. The method of any one of the preceding claims, wherein the at least one part of the dose event record, for each dose event, comprises information relating to at least one of, an arbitrary selected plurality of, or all of a unique dose event identifier, a dose quantity and $T_{Stamp}(e)$ of the recording unit (11).

4. The method of any one of the preceding claims, wherein the recording unit comprises a timer configured to provide time information for the recording unit (11), the time information being $T_{RTC}$.

5. The method of any one of the preceding claims, further comprising the step of defining (S101) a determination period in the at least one part of the dose event record, wherein the determination period comprises one or more dose events ($e_i$ to $e_k$) for which $T_{Dose}(e)$ is to be determined, wherein $k \geq i \geq 1$, and wherein the absolute time $T_{Dose}(e)$ of a particular dose event ($e$) of the one or more dose events ($e_i$ to $e_k$) within the determination period, is determined using a determination function.

6. The method of claim 5 with additional reference to claim 2, wherein the determination function $f$ is a function of the time information from the recording unit (11) and the time information from the receiving unit (71), such that:

$$T_{Dose}(e) = f(T_{Client}, T_{RTC\_trans}, T_{Stamp}(e)),$$

or such that

$$T_{Dose}(e) = f(T_{Client}, (T_{RTC\_trans} - T_{Stamp}(e))),$$

or such that

$$T_{Dose}(e) = f(T_{Client} - (T_{RTC\_trans} - T_{Stamp}(e))),$$

or such that

$$T_{Dose}(e) = T_{Client} - (T_{RTC\_trans} - T_{Stamp}(e)).$$

7. The method of any one of the preceding claims, further comprising the step of evaluating (S102) the at least one part of the dose event record with respect to an occurrence of an irregularity in a time information contained in the at least one part of the dose event record, wherein the evaluation is based on the information stored in the dose event record and/or time information from the recording unit (11) and/or time information from the receiving unit (71).

8. The method of claim 7, wherein the irregularity is a reset event of the recording unit (11) at which the timer of the recording unit (11) is reset, resulting in a change of $T_{RTC}$ to a default value of $T_{RTC}$, e.g. zero.

9. The method of claim 7 or 8, wherein the occurrence of an irregularity is evaluated based on a detection of a change in a variable $\Delta$, wherein $\Delta$ is a function of time information from the recording unit (11) and time information from the receiving unit (71), e.g. wherein $\Delta$ is a function of $T_{RTC\_m}$ and $T_{Client\_p}$, preferably $\Delta = T_{Client\_p} - T_{RTC\_m}$, wherein

a) $T_{RTC\_m}$ is a transmission related time information of the recording unit (11) which is indicative for the time ($m$) at which the at least one part of the dose event record is transmitted to be received by the receiving unit (71); and

b) $T_{Client\_p}$ is a time information from the receiving unit (71) which is indicative for the time (p) at which the at least one part of the dose event record is received by the receiving unit (71).

10. The method of any one of claims 7 to 9 with additional reference to claim 5, wherein the determination function for the particular dose event ($e$) is selectable or selected (S103) from a plurality of different determination functions depending on the occurrence of the irregularity in the time information of the at least one part of the dose event record during the determination period wherein

a) a determination function, $g$, is selected if the determination period does not contain an irregularity or if the dose event for which $T_{Dose}(e)$ is to be determined was recorded after a last reset of $T_{RTC}$ in the determination period, wherein the last reset of $T_{RTC}$ is a reset of $T_{RTC}$ in the determination period after which there is no subsequent reset of $T_{RTC}$ in the determination period; and/or
b) a determination function, $h$, is selected if the determination period starts after a time (p) at which at least a part of the dose event record had been received by the receiving unit and further if the dose event ($e$) for which $T_{Dose}(e)$ is to be determined was recorded before a first reset of $T_{RTC}$ within the determination period; and/or
c) a determination function, $i$, is selected in any other case, e.g. if the dose event for which $T_{Dose}(e)$ is to be determined was recorded between an $n$-th reset of $T_{RTC}$ and an ($n$ + 1)-th reset of $T_{RTC}$ in the determination period, with $n \geq 1$.

11. The method of claim 10, wherein

$$T_{Dose}(e) = g(T_{Client} - (T_{RTC\_trans} - T_{Stamp}(e))),$$

and/or

$$T_{Dose}(e) = h(\Delta_{pre} + T_{Stamp}(e)),$$

and/or

$$T_{Dose}(e) = i(T_{User}(e_{other}) - T_{Stamp}(e_{other}) + T_{Stamp}(e)),$$

wherein

a) $T_{Stamp}(e)$ is a dose event related time information of the recording unit (11) which is indicative for the time at which the dose event ($e$) was recorded in the dose event record;
b) $T_{RTC\_trans}$ is a transmission related time information of the recording unit (11) which is indicative for the time at which the at least one part of the dose event record is transmitted to be received by the receiving unit (71);
c) $T_{RTC\_m}$ is a transmission related time information of the recording unit (11) which is indicative for the time ($m$) at which the at least one part of the dose event record is transmitted to be received by the receiving unit (71);
d) $T_{Client}$ is a time information from the receiving unit (71) which is indicative for the time at which the at least one part of the dose event record is received by the receiving unit (71);
e) $T_{Client\_p}$ is a time information from the receiving unit (71) which is indicative for the time (p) at which the at least one part of the dose event record is received by the receiving unit (71);
f) $\Delta_{pre}$ is a variable indicative for a time deviation between the recording unit (11) and the receiving unit (71) which is determined based on time information from the recording unit (11) and time information from the receiving unit (71), e.g. based on $T_{RTC\_m}$ and $T_{Client\_p}$;
g) $T_{User}(e_{other})$ is a user entered time of one other dose event ($e_{other}$) which was recorded in the dose event record in a same portion of the determination period as the dose event ($e$);
h) $T_{Stamp}(e_{other})$ is a dose event related time information of the recording unit (11) which is indicative for the time at which the other dose event ($e_{other}$) was recorded in the dose event record; and

wherein $g$, h and $i$ are functions of the variables and/or expressions specified in the brackets.

12. The method of claim 7 or 8 with additional reference to claim 5, wherein the determination function for the particular dose event ($e$) is selectable or selected (S103) from a plurality of different determination functions depending on the occurrence of the irregularity in the time information of the at least one part of the dose event record during the determination period and/or depending on whether the determination period starts with a reset of $T_{RTC}$, wherein

a) a determination function, $g$, is selected if the determination period starts with a reset of $T_{RTC}$ and does not contain any irregularity or if the dose event for which $T_{Dose}(e)$ is to be determined was recorded after a last reset of $T_{RTC}$ in the determination period, wherein the last reset of $T_{RTC}$ is a reset of $T_{RTC}$ in the determination period after which there is no subsequent reset of $T_{RTC}$ in the determination period; and/or

b) a determination function, $j$, is selected, if the dose event for which $T_{Dose}(e)$ is to be determined was recorded between a first dose event recorded in the dose event record and a first irregularity in the determination period; and/or

c) a determination function, $k$, is selected, if there is no reset of $T_{RTC}$ in the determination period; and/or

d) a determination function, $l$, is selected, if the determination period starts after a time (p) at which at least one part of the dose event record had been successfully received by the receiving unit (71), and

if the dose event (e) for which $T_{Dose}(e)$ is to be determined was recorded before a first reset of $T_{RTC}$ within the determination period,

wherein the first reset of $T_{RTC}$ is a reset in the determination period before which there is no previous reset of $T_{RTC}$ in the determination period.

**13.** The method of claim 12, wherein

$$T_{Dose}(e) = g(T_{Client} - (T_{RTC\_trans} - T_{Stamp}(e))),$$

and/or

$$T_{Dose}(e) = j(T_{RTC\_FDE} + T_{Stamp}(e)),$$

and/or

$$T_{Dose}(e) = k(T_{Client\_p+1} - (T_{RTC\_m+1} - T_{Stamp}(e)) * CF),$$

and/or

$$T_{Dose}(e) = l(T_{Client\_p} - (T_{RTC\_m} - T_{Stamp}(e))),$$

wherein

a) $T_{Stamp}(e)$ is a dose event related time information of the recording unit (11) which is indicative for the time at which the dose event (e) was recorded in the dose event record;

b) $T_{RTC\_trans}$ is a transmission related time information of the recording unit (11) which is indicative for the time at which the at least one part of the dose event record is transmitted to be received by the receiving unit (71);

c) $T_{RTC\_m}$ is a transmission related time information of the recording unit (11) which is indicative for a first point in time (m) at which the at least one part of the dose event record is transmitted to be received by the receiving unit (71);

d) $T_{RTC\_FDE}$ is a first dose event time information of the recording unit (11) which is indicative for the time at which a first dose event is recorded in the dose event record;

e) $T_{RTC\_m+1}$ is a transmission related time information of the recording unit (11) which is indicative for a second point in time (m + 1) at which at least a second part of the dose event record is transmitted to be received by the receiving unit (71), the second point in time (m + 1) being a point in time after the first point in time (m) at which a first part of the dose event record had been transmitted to be received by the receiving unit;

f) $T_{Client}$ is a time information from the receiving unit (71) which is indicative for the time at which the at least one part of the dose event record is received by the receiving unit (71);

g) $T_{Client\_p}$ is a time information from the receiving unit (71) which is indicative for a third point in time (p) at which the at least one part of the dose event record is received by the receiving unit (71);

h) $T_{Client\_p+1}$ is a time information of the receiving unit (71) which is indicative for a fourth point in time (p + 1)

at which the second part of the dose event record is received by the receiving unit (71), the fourth point in time (p + 1) being a point in time after a third point in time (p) at which the first part of the dose event record had been received by the receiving unit;

i) *CF* is a correction factor, which is a function of time information from the recording unit (11) and of time information from the receiving unit (71), e.g.

$$CF = 1 - \frac{(T_{RTC\_m+1} - T_{RTC\_m}) - (T_{Client\_p+1} - T_{Client\_p})}{(T_{RTC\_m+1} - T_{RTC\_m})};$$

and

wherein *g*, *j*, *k*, and *l* are functions of the variables and/or expressions specified in the brackets.

14. The method of any one of the preceding claims, wherein

a) prior to the determination of $T_{Dose}(e)$, the method comprises the step of transmitting (S90) the at least one part of the dose event record from the recording unit (11) to the receiving unit (71), and/or wherein
b) prior to the transmission (S90) of the at least one part of the dose event record, the method comprises the step of recording (S80) information relating to one or more dose events ($e_1$ to $e_z$) performed with a medical device with a recording unit (71) in the dose event record.

15. A dose event processing device (70) for determining an absolute time, $T_{Dose}(e)$, of one or more recorded dose events ($e_1$ to $e_z$) performed with a medical device, wherein e characterizes one particular dose event of the one or more dose events, the dose event processing device (70) comprising:

a receiving unit (71) configured to receive at least a part of a dose event record; and
a processing unit (72),
wherein the dose event processing device (70) is configured to perform the method according to any one of claims 1 to 14.

16. A computer-readable storage medium, e.g. a non-transitory computer-readable storage medium, having stored thereon a computer program product comprising machine-readable instructions which, when the instructions are executed by the processing unit (72), causes the dose event processing device (70) according to claim 15 to carry out the method according to any one of claims 1 to 14, or controls the method according to any one of claims 1 to 14.

Fig. 1

Fig. 2

Fig. 3

## Fig. 4

## Fig. 5

## Fig. 6

## Fig. 7

## Fig. 8

EP 4 350 703 A1

Fig. 9

30

| Europäisches Patentamt European Patent Office Office européen des brevets | **EUROPEAN SEARCH REPORT** | Application Number EP 22 31 5233 |
|---|---|---|

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/163793 A1 (KOEHLER MATTHIAS [DE] ET AL) 25 June 2009 (2009-06-25) * figure 1 * * page 1, paragraph 0002 – page 3, paragraph 0033 * * page 5, paragraph 0044 – page 6, paragraph 0055 * * page 8, paragraph 0072 – paragraph 0080 * ----- | 1-16 | INV. G16H15/00 G16H20/17 ADD. G16H10/60 A61M5/14 |
|  |  |  | TECHNICAL FIELDS SEARCHED (IPC) G16H A61M |

The present search report has been drawn up for all claims

| Place of search | Date of completion of the search | Examiner |
|---|---|---|
| Munich | 20 February 2023 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 31 5233

This annex lists the patent family members relating to the patent documents cited in the above-mentioned European search report.
The members are as contained in the European Patent Office EDP file on
The European Patent Office is in no way liable for these particulars which are merely given for the purpose of information.

20-02-2023

| Patent document cited in search report | | Publication date | Patent family member(s) | | Publication date |
|---|---|---|---|---|---|
| US 2009163793 | A1 | 25-06-2009 | CA | 2645944 A1 | 21-06-2009 |
| | | | CN | 101461969 A | 24-06-2009 |
| | | | DK | 2073135 T3 | 02-01-2019 |
| | | | EP | 2073135 A1 | 24-06-2009 |
| | | | HK | 1129612 A1 | 04-12-2009 |
| | | | JP | 5404021 B2 | 29-01-2014 |
| | | | JP | 2009183692 A | 20-08-2009 |
| | | | US | 2009163793 A1 | 25-06-2009 |

EPO FORM P0459

For more details about this annex : see Official Journal of the European Patent Office, No. 12/82